# FASCICULE DE BREVET EUROPEEN

(11) **EP 0 769 290 B1**
(45) Date de publication et mention de la délivrance du brevet: **19.12.2001**
(21) Numéro de dépôt: 96401859.2
(22) Date de dépôt: 29.08.1996
(51) Int. Cl.: A61K 7/06, A61K 7/48

(54) **Composition topique comprenant un polymère à greffons siliconés et un polymère amphiphile à chaîne grasse**
Topische Zusammensetzung, die ein Polymer mit Siliconpfropfzweigen und ein amphiphiles Polymer mit Fettkette enthält.
Topical composition containing a silicon grafted polymer and an amphiphilic polymer with a fatty chain

(30) Priorité: 29.09.1995 FR 9511483
(43) Date de publication de la demande: 23.04.1997
(73) Titulaire: L'OREAL, 75008 Paris (FR)
(72) Inventeur: Dubief, Claude, 78150 Le Chesnay (FR); Dupuis, Christine, 75018 Paris (FR)
(74) Mandataire: Miszputen, Laurent

(56) Documents cités:
- EP-A- 0 412 706
- EP-A- 0 524 612
- WO-A-91/15186
- WO-A-95/00108
- FR-A- 2 709 955

## Description

La présente invention a trait à une composition cosmétique ou dermatologique pour le traitement des matières kératiniques, en particulier des cheveux humains, comprenant au moins un polymère siliconé greffé à squelette organique non-siliconé greffé par des monomères contenant un polysiloxane et au moins un polymère amphiphile ionique comportant au moins une chaîne grasse et au moins un motif hydrophile.

Les polymères du type polymère siliconé à squelette organique non-siliconé greffé par des monomères contenant un polysiloxane sont connus dans l'art antérieur pour leurs propriétés coiffantes. Ils sont particulièrement intéressants en cosmétique capillaire du fait qu'ils apportent de la tenue aux cheveux. Leurs propriétés cosmétiques après application sur les cheveux sont néanmoins insuffisantes. On constate que les cheveux présentent après application de ces polymères, un toucher rêche et crissant résultant d'une répartition discontinue du polymère le long des fibres des cheveux.

La demanderesse a constaté que certains agents épaississants classiques tels que, par exemple les homopolymères poly(acide acrylique) réticulés, utilisés dans des compositions capillaires contenant ces polymères particuliers avaient tendance à diminuer la viscosité de la composition et ne permettaient pas d'améliorer sensiblement la répartition de la composition le long des fibres de cheveux mouillés ou séchés ni d'améliorer sensiblement les propriétés de douceur au toucher ou de démêlage, après application.

La demande internationale WO95/00108 décrit des compositions cosmétiques stables et agréables au toucher comprenant un polymère à squelette organique greffé par des macromères siliconés et une résine fixante non siliconée. Toutefois, ce document est muet sur l'augmentation de la viscosité et la répartition de la composition le long de la fibre capillaire.

La demanderesse a trouvé de façon surprenante que l'utilisation d'un polymère amphiphile ionique comportant au moins une chaîne grasse et au moins un motif hydrophile comme agent épaississant dans des compositions capillaires contenant un polymère à squelette organique non-siliconé greffé par des monomères contenant un polysiloxane, permettait non seulement d'augmenter de façon accrue la viscosité du milieu de ces compositions mais aussi d'améliorer, à l'application, le dépôt du polymère siliconé greffé le long des fibres kératiniques et d'améliorer leurs propriétés cosmétiques notamment au niveau du toucher ainsi qu'au niveau du démêlage, tout en conservant les propriétés coiffantes du polymère siliconé greffé.

La composition selon l'invention est donc essentiellement caractérisée par le fait qu'elle contient dans un milieu cosmétiquement ou dermatologiquement acceptable au moins un polymère siliconé greffé à squelette organique non-siliconé greffé par des monomères contenant un polysiloxane et au moins un polymère amphiphile ionique comportant au moins une chaîne grasse et et au moins un motif hydrophile.

Dans ce qui suit, on entend désigner par silicone ou polysiloxane, en conformité avec l'acceptation générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Dans ce qui suit, on entend désigner par «macromère polysiloxane», en conformité avec l'acceptation générale, tout monomère contenant dans sa structure une chaîne polymère du type polysiloxane.

Les polymères siliconés conformes à la présente invention sont constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

Les monomères organiques non-siliconés constituant la chaîne principale du polymère siliconé greffé peuvent être choisis parmi des monomères à insaturation éthylènique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères siliconés greffés de l'invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés convenant pour la réalisation de la présente invention est constituée par les copolymères greffés siliconés comprenant :
a) de 0 à 98% en poids d'au moins un monomère(A) lipophile de faible polarité lipophile à insaturation éthylénique, polymérisable par voie radicalaire ;
b) de 0 à 98% en poids d'au moins un monomère (B) hydrophile polaire à insaturation éthylénique, copolymérisable avec le ou les monomères du type (A)
c) de 0,01 à 50 % en poids d'au moins un macromère polysiloxane (C) de formule générale :

   X(Y)ₙSi(R)₃₋ₘZₘ (I)
où :
X désigne un groupe vinylique copolymérisable avec les monomères (A) et (B) ;
Y désigne un groupe de liaison divalent ;
R désigne un hydrogène, un alkyle ou un alcoxy en C₁-C₆, un aryle C₆-C₁₂ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,693,935, US 4,728,571 et US 4,972,037 et les demandes de brevet EP-A-0412704, EP-A-0412707, EP-A-0640105. Ils ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertiobutylstyrène ; le butadiène ; le cyclohexadiène ; l'éthylène ; le propylène ; le vinyltoluène ; les esters d'acide acrylique ou méthacrylique et de 1,1-dihydro-perfluoroalcanol ou de ses homologues ; les esters d'acide acrylique ou méthacrylique et de oméga-hydrydofluroalcanol; les esters d'acide acrylique ou méthacrylique et de fluoroalkylsulfoamido-alcool ; les esters d'acide acrylique ou méthacrylique et d'alcool fluoroalkylique ; les esters d'acide acrylique ou méthacrylique et de fluroéther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perflurooctane sulfonamido)-éthylacrylate ; le 2-(N-butylperflurooctane sulfonamido)-éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quatemisé, le (méth)acrylamide, le N-t-butylacrylamide, l'acide maleique, l'anhydride maléique et leurs demi-esters, les (méth) acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quatemisé, la vinylpyrrolidone et leurs mélanges.

Les macromères polysiloxane (C) de formule (I) préférentiels sont choisis parmi ceux répondant à la formule générale suivante (II) : 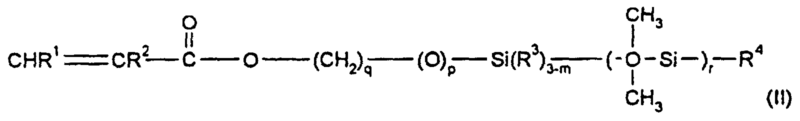 dans laquelle :
R¹ est hydrogène ou -COOH (de préférence hydrogène) ;
R² est hydrogène, méthyle ou -CH₂COOH (de préférence méthyle) ;
R³ est alkyle, alcoxy ou alkylamino en C₁-C₆, aryle en C₆-C₁₂ ou hydroxyle (de préférence méthyle) ;
R⁴ est alkyle, alcoxy ou alkylamino en C₁-C₆, aryle en C₆-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1).

On utilise plus particulièrement les macromères polysiloxanes de formule : 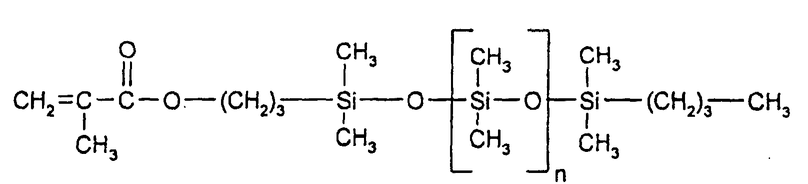 avec n étant un nombre allant de 5 à 700.

Un mode particulier de réalisation de l'invention consiste à utiliser un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 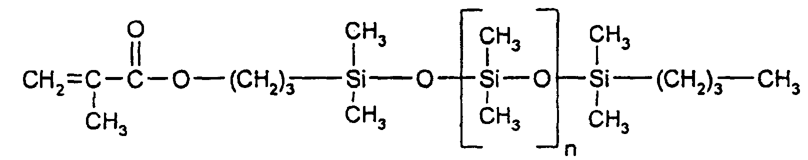 avec n étant un nombre allant de 5 à 700 ; les pourcentages en poids étant calculés par rapport au poids total des monomères.

Un autre mode particulier de réalisation de l'invention consiste à utiliser un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 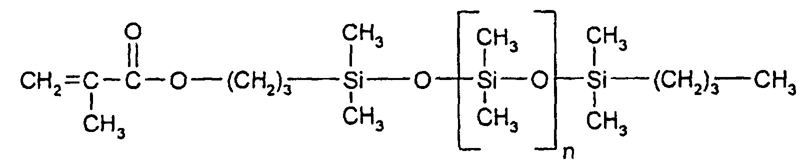 avec n étant un nombre allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaîne principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkylstyrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxylique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (III):

T-(CH₂)ₛ-Si-[-(OSiR⁵R⁶)ₜ-R⁷]_{y} (III)

dans laquelle T est choisi dans le groupe constitué par NH2, NHR', une fonction époxy, OH, SH ; R⁵, R⁶, R⁷ et R', indépendamment, désignent un alkyle en C₁-C₆, phenyle, benzyle, ou alkylphényle en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9.000 à 40.000.

Les polymères greffés siliconés de l'invention sont utilisés de préférence en une quantité allant de 0,01 à 15% en poids du poids total de la composition. Plus préférentiellement, cette quantité varie de 0,5 à 10% en poids.

Les polymères amphiphiles ioniques comportant au moins une chaîne grasse et des motifs hydrophiles, utilisés selon l'invention, sont choisis de préférence dans le groupe constitué par :
(1) les holosides ioniques modifiés par des groupements comportant au moins une chaîne grasse ;
   on peut citer à titre d'exemple :
   - les celluloses cationiques quaternisées modifiées par des groupements comportant au moins une chaîne grasse tels que des groupes alkyle, arylalkyle, alkylaryle ou leurs mélanges où les groupes alkyle sont de préférence en C₈-C₂₂ ;
   - les alkylhydroxyéthylcelluloses quaternisées (cationiques) telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) vendus par la société AMERCHOL et les produits CRODACEL QM, CRODACEL QL (alkyle en C₁₂) et CRODACEL QS (alkyle en C₁₈) vendus par la société CRODA ;
   - les polyalcools (C₁₂-C₁₈) saccharides anioniques tels que le produit EMULSAN (mélange D-galactosamine/acide aminouronique) vendu par la société PETROFERM ;
(2) les copolymères d'anhydride maléique et de monomères comportant au moins une chaîne grasse ;
   on peut citer à titre d'exemple :
   - les copolymères N-octadécylvinylétherlanhydride maléique comme le produit GANTREZ AN-8194 vendu par la société ISP ;
(3) les copolymères de l'acide crotonique et de monomères comportant au moins une chaîne grasse ;
   on peut citer à titre d'exemple :
   - les terpolymères acétate de vinyle/acide crotonique/stéarate d'allyle ;
(4) les copolymères d'acide (méth)acrylique et de monomères comportant au moins une chaîne grasse ; ces monomères sont choisis parmi les monomères hydrophobes à chaîne grasse, les monomères amphiphiles comportant une partie hydrophobe à chaîne grasse et une partie hydrophile ou bien leurs mélanges ;
   on peut citer à titre d'exemple :
   - les copolymères réticulés d'acide acrylique/acrylate d'alkyle en C₁₀-C₃₀ tels que les produits PEMULEN TR 1, PEMULEN TR 2, CARBOPOL 1382, CARBOPOL 1342 et CARBOPOL ETD 2020 vendus par la société GOODRICH ;
   - les copolymères acide (méth)acrylique/acrylate d'éthyle/acrylate d'alkyle tels que le produit ACUSOL 823 vendu par la société ROHM & HAAS et le produit IMPERON R vendu par la société HOECHST ;
   - les copolymères réticulés acide acrylique/isodécanoate de vinyle tel que le produit STABYLEN 30 vendu par la société 3V ;
   - les terpolymères acide acrylique/vinylpyrrolidone/méthacrylate de lauryle tels que les produits ACRYLIDONE LM, ACP-1184, ACP-1194 vendus par la société ISP;
   - les copolymères acide acrylique/(méth)acrylate de lauryle tels que les produits COATEX SX vendus par la société COATEX ;
   - les terpolymères acide (méth)acrylique/acrylate d'alkyle/alkyl polyéthoxylé allyl éther tels que les produits RHEOVIS -CR, -CR3, -CR2 et -CRX vendus par la société ALLIED COLLOIDS ;
   - les terpolymères acide méthacrylique/acrylate d'éthyle/stéaryl polyéthoxylé allyl éther tels que les produits SALCARE-SC90 et -SC80 vendus par la société ALLIED COLLOIDS (stéaryl polyéthoxylé à 10 moles d'oxyde d'éthylène noté stéareth-10) ;
   - les terpolymères acide méthacrylique/acrylate d'éthyle/acrylate de lauryle polyoxyéthyléné tels que le produit RHEO 2000 vendu par COATEX ;
   - les terpolymères acide méthacrylique/acrylate d'éthyle/méthacrylate de stéaryle polyoxyéthyléné tels que les produits ACRYSOL 22, ACRYSOL25 et DW-1206A vendus par la société ROHM & HAAS ;
   - les copolymères acide méthacryliquelacrylate d'éthyle/acrylate de nonylphénol polyoxyéthyléné tels que le produit RHEO 3000 vendu par COATEX ;
   - les copolymères acide acrylique/monoitaconate de stéaryle polyoxyéthyléné ou les copolymères acide acrylique/monoitaconate de cétyle polyoxyéthyléné tels que les produits 8069-72A et 8069-72B vendus par NATIONAL STARCH ;
   - les copolymères acide méthacrylique/acrylate de butyle/monomère hydrophobe comportant une chaîne grasse tels que le produit 8069-146A vendu par NATIONAL STARCH;
   - les terpolymères acide acrylique/acrylate d'alkyle en C₁₅/acrylate de polyéthylèneglycol (28 moles d'oxyde d'éthylène) tels que le produit DAPRAL GE 202 vendu par la société AKZO ;
   - les sels d'un ester d'acide gras partiel d'un copolymère acide acrylique/diméthyléthanolamine tels que le produit DAPRAL GE 202 DMA vendu par la société AKZO ;
   - les copolymères acide acryliquelacrylate/monomère amphiphile comportant une chaîne grasse à groupements uréthane tels que le produit ADDITOL VXW 1312 vendu par HOECHST ;
   - les copolymères acryliques modifiés par des groupes hydrophobes à chaîne grasse tels que le produit ACUSOL 102 vendu par ROHM & HAAS.

Les polymères amphiphiles comportant au moins une chaîne grasse et au moins motif hydrophile selon l'invention, sont utilisés de préférence en une quantité comprise entre 0,01 et 20% en poids du poids total de la composition. Plus préférentiellement, cette quantité varie de 0,1 à 15% en poids et encore plus préférentiellement de 0,5 à 10% en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.
Les polymères siliconés greffés selon l'invention peuvent être dissous dans ledit mileu cosmétiquement acceptable ou utilisés sous forme de dispersion aqueuse de particules.

La composition de l'invention peut également contenir au moins un additif choisi parmi les épaississants sans chaîne grasse, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement non-thérapeutique des matières kératiniques telles que les cheveux humains consistant à appliquer sur celles-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits.

### EXEMPLES

### EXEMPLE 1 Gel de coiffage

- Polymère greffé siliconé de structure (1) telle que définie ci-dessous 0,5 g en MA
- Terpolymère acide méthacrylique/acrylate d'éthyle/méthacrylate de stéaryle oxyéthyléné (55/35/10) en dispersion aqueuse à 30% vendu sous le nom ACRYSOL 22 par la Société ROHM & HAAS 1 g en MA
- Aminométhylpropanol neutralisation à 100% dudit polymère siliconé et du terpolymère qsp
- Eau déminéralisée qsp 100 g

### Structure (1):

Copolymère obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 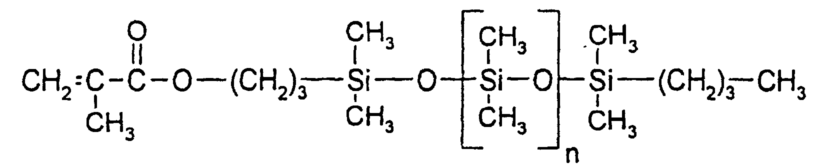 avec n étant un nombre choisi de telle sorte que le poids moléculaire moyen en nombre du macromère soit compris entre environ 9.000 et 12.000 ; les pourcentages en poids étant calculés par rapport au poids total des monomères.

### ESSAIS COMPARATIFS DE VISCOSITE

On étudie les propriétés rhéologiques d'un agent épaississant classique du type homopolymère poly(acide acrylique) réticulé comme le produit SYNTHALEN K vendu par la société 3V dans une solution aqueuse contenant 1% en poids de cet épaississant et dans une solution aqueuse contenant 1% en poids de cet épaississant et 1% en poids de polymère siliconé greffé tel que celui décrit dans l'exemple 1. On mesure les viscosités des solutions épaissies au moyen d'un appareil RHEOMAT180 équipé du système CONTRAVES TV. Toutes les solutions sont neutralisées à pH 7,5 par de l'aminométhylpropanol.

On étudie les propriétés rhéolologiques d'un polymère P₁ amphiphile ionique comportant une chaîne grasse et au moins un motif hydrophile selon l'invention dans une solution aqueuse contenant 1% en poids de ce polymère amphiphile épaississant et dans une solution aqueuse contenant 1% en poids de cet épaississant et 1% en poids de polymère siliconé greffé tel que celui décrit dans l'exemple 1.

Le polymère **P**_{**1**} amphiphile selon l'invention qui a été étudié est le suivant :
- **P**_{**1**} : Terpolymère acide méthacrylique/acrylate d'éthyle/méthacrylate de stéaryle oxyéthyléné (55/35/10) en dispersion aqueuse à 30% vendu sous le nom ACRYSOL 22 par la Société ROHM & HAAS ;

Les viscosités des solutions sont indiquées en centipoises dans le tableau suivant :

| **Polymère étudié** | **Viscosité en cps de la solution aqueuse contenant 1% d'agent épaississant seul ou le polymère siliconé greffé seul** | **Viscosité en cps de la solution aqueuse contenant 1% en poids d'agent épaississant et 1% en poids de polymère greffé siliconé de l'exemple 1** |
|---|---|---|
| Polymère siliconé greffé de l'exemple 1 | 40 | pas d'épaississant |
| Homopolymère poly(acide acrylique) réticulé | 7500 | 3580 |
| **P**_{**1**} | 1700 | 14.000 |

On constate que l'homopolymère poly(acide acrylique) réticulé en association avec le polymère greffé siliconé de l'invention diminue la viscosité de la formulation tandis que le polymère épaississant **P**_{**1**} amphiphile ionique selon l'invention, comportant une chaîne grasse et au moins un motif hydrophile augmente sensiblement la viscosité de la solution contenant le polymère greffé siliconé.

### ESSAIS COMPARATIFS SUR LES PROPRIETES COSMETIQUES

On effectue un test d'estimation sensorielle sur un panel de 5 personnes. On étudie comme critères cosmétiques le démêlage et la douceur au toucher après application sur des mèches de cheveux, mouillées et sensibilisées du type SA 20. On applique sur chacune de ces 5 personnes, sur des mèches préalablement lavées au shampooing, les trois solutions A, B et C suivantes à une dose de 0,5 g pour 5 g de mèche :
Solution A contenant 1% en poids du polymère siliconé greffé de l'exemple 1 ;
Solution B contenant 1% en poids du polymère siliconé greffé de l'exemple 1 et 1% en poids de l'homopolymère poly(acide acrylique) réticulé SYNTHALEN K
Solution C contenant 1% en poids du polymère siliconé greffé de l'exemple 1 et 1% en poids de polymère **P**_{**1**} tel que décrit ci-dessus.

Toutes les solutions A, B et C sont neutralisées à pH 7,5 par de l'aminométhylpropanol.

Pour chaque critère cosmétique, les personnes testées attribuent une note d'appréciation de 0 à 5. Les résultats des tests sont résumés dans le tableau ci-dessous.

| **Solutions testées** | **Appréciation du démêlage** | **Appréciation de la douceur au toucher** |
|---|---|---|
| A | 2 | 1,5 |
| B | 3 | 2,5 |
| C | 3,5 | 3 |

Les 5 personnes interrogées ont estimé que la présence du polymère amphiphile ionique épaississant à chaîne grasse et au moins un motif hydrophile selon l'invention dans la solution C contenant le polymère siliconé greffé améliorait la douceur des cheveux au toucher et le démêlage des cheveux par rapport à la solution A contenant le polymère siliconé greffé seul ou la solution B contenant ledit polymère siliconé greffé en association avec l'épaississant classique du type homopolymère poly(acide acrylique) réticulé.

## Revendications

1. Composition cosmétique ou dermatologique destinée au traitement des matières kératiniques, **caractérisée par le fait qu'**elle contient, dans un milieu cosmétiquement ou dermatologiquement acceptable, au moins un polymère siliconé greffé à squelette organique non-siliconé greffé par des monomères contenant un polysiloxane et au moins un polymère amphiphile ionique comportant au moins une chaîne grasse et au moins un motif hydrophile, choisi parmi :
- les holosides ioniques modifiés par des groupements comportant au moins une chaîne grasse ;
- les copolymères d'anhydride maléique et de monomères comportant au moins une chaîne grasse ;
- les terpolymères acétate de vinyle/acide crotonique/stéarate d'allyle;
- les copolymères réticulés acide acrylique/isodécanoate de vinyle ;
- les terpolymères acide acrylique/vinylpyrrolidone/méthacrylate de lauryle;
- les copolymères acide acrylique/(méth)acrylate de lauryle ;
- les terpolymères acide méthacrylique/acrylate d'éthyle/stéaryl polyéthoxylé allyl éther;
- les terpolymères acide méthacrylique/acrylate d'éthyle/acrylate de lauryle polyoxyéthyléné;
- les terpolymères acide méthacrylique/acrylate d'éthyle/méthacrylate de stéaryle polyoxyéthyléné ;
- les copolymères acide méthacrylique/acrylate d'éthylelacrylate de nonylphénol polyoxyéthyléné;
- les copolymères acide acrylique/monoitaconate de stéaryle polyoxyéthyléné ;
- les copolymères acide acrylique/monoitaconate de cétyle polyoxyéthyléné;
- les copolymères acide méthacrylique/acrylate de butyle/monomère hydrophobe comportant une chaîne grasse;
- les terpolymères acide acryliquelacrylate d'alkyle en C₁₅/acrylate de polyéthylèneglycol (28 moles d'oxyde d'éthylène) ;
- les sels d'un ester d'acide gras partiel d'un copolymère acide acrylique/diméthyléthanolamine;
- les copolymères acide acrylique/acrylate/monomère amphiphile comportant une chaîne grasse à groupements uréthane.

2. Composition selon la revendication 1, **caractérisée par le fait que** le polymère siliconé greffé est constitué d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane.

3. Composition selon la revendication 2, **caractérisée par le fait que** les monomères organiques non-siliconés constituant la chaîne principale du polymère siliconé greffé sont choisis dans le groupe constitué par des monomères à insaturation éthylénique polymérisables par voie radicalaire, des monomères polymérisables par polycondensation, des monomères à ouverture de cycle.

4. Composition selon l'une quelconque des revendications 1 à 3, **caractérisée par le fait que** le polymère siliconé greffé est un copolymère greffé siliconé comprenant:
a) de 0 à 98% en poids d'au moins un monomère(A) lipophile de faible polarité à insaturation éthylénique de faible polarité, polymérisable par voie radicalaire ;
b) de 0 à 98% en poids d'au moins un monomère (B) hydrophile polaire à insaturation éthylénique, copolymérisable avec le ou les monomères du type (A) ;
c) de 0,01 à 50 % en poids d'au moins un macromère polysiloxane (C) de formule générale :
X(Y)ₙSi(R)₃₋ₘ Zₘ (I)
où :
X désigne un groupe vinylique copolymérisable avec les monomères (A) et (B) ;
Y désigne un groupe de liaison divalent ;
R désigne un hydrogène, un alkyle ou un alcoxy en C₁-C₆, un aryle C₆-C₁₂ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

5. Composition selon la revendication 4, **caractérisée par le fait que** les monomères lipophiles (A) sont choisis dans le groupe constitué par les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluoroalcanol ; les esters d'acide acrylique ou méthacrylique et de fluoroalkylsulfoamido-alcool; les esters d'acide acrylique ou méthacrylique et d'alcool fluoroalkylique ; les esters d'acide acrylique ou méthacrylique et de fluoroéther d'alcool ; ou leurs mélanges.

6. Composition selon la revendication 5, **caractérisée par le fait que** les monomères lipophiles (A) sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-butylperfluorooctane sulfonamido)-éthylacrylate, le 2-(N-méthylperfluorooctane sulfonamido)-éthylacrylate et leurs mélanges.

7. Composition selon l'une quelconque des revendications 4 à 6, **caractérisée par le fait que** les monomères polaires (B) sont choisis dans le groupe constitué par l'acide acrylique, l'acide méthacrylique, le N,N-diméthylacrylamide, le méthacrylate de diméthylaminoéthyle, le méthacrylate de diméthylaminoéthyle quatemisé, le (méth)acrylamide, le N-t-butylacrylamide, l'acide maleique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinyl caprolactame ou leurs mélanges.

8. Composition selon la revendication 7, **caractérisée par le fait que** les monomères polaires (B) sont choisis dans le groupe constitué par l'acide acrylique, le N,N-diméthylacrylamide, le méthacrylate de diméthylaminoéthyle, le méthacrylate de diméthylaminoéthyle quatemisé, la vinylpyrrolidone et leurs mélanges.

9. Composition selon l'une quelconque des revendications 4 à.8, **caractérisée par le fait que** le macromère polysiloxane (C) correspond à la formule générale suivante (II) : 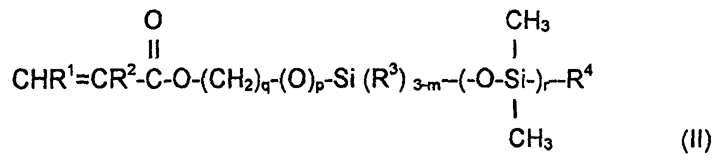 dans laquelle :
R¹ est hydrogène ou -COOH ;
R² est hydrogène, méthyle ou -CH₂COOH ;
R³ est alkyle, alcoxy ou alkylamino en C₁-C₆, aryle en C₆-C₁₂ ou hydroxyle ;
R⁴ est alkyle, alcoxy ou alkylamino en C₁-C₆, aryle en C₆-C₁₂ ou hydroxyle ;
q est un entier de 2 à 6 ;
p est 0 ou 1 ;
r est un nombre entier de 5 à 700 ;
m est un entier allant de 1 à 3 ;

10. Composition selon l'une quelconque des revendications 4 à 9, **caractérisée par le fait que** le macromère polysiloxane (C) correspond à la formule générale suivante : 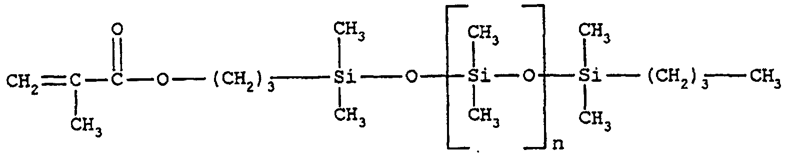 avec n étant un nombre allant de 5 à 700 ; les pourcentages en poids étant calculés par rapport au poids total des monomères.

11. Composition selon l'une quelconque des revendications 1 à 10, **caractérisée par le fait que** le polymère siliconé greffé est un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 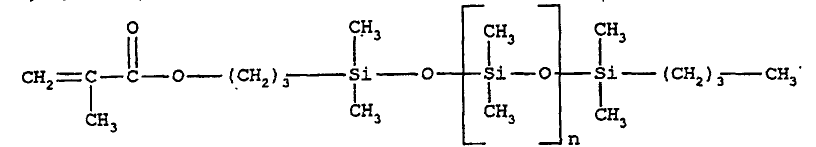 avec n étant un nombre allant de 5 à 700 ; les pourcentages en poids étant calculés par rapport au poids total des monomères.

12. Composition selon l'une quelconque des revendications 1 à 10, **caractérisée par le fait que** le polymère siliconé greffé est un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 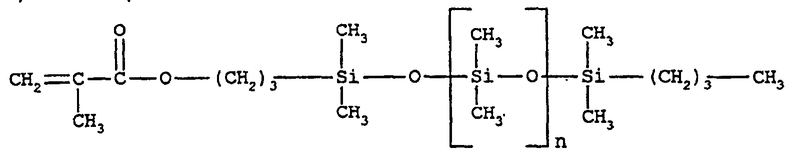 avec n étant un nombre allant de 5 à 700 ; les pourcentages en poids étant calculés par rapport au poids total des monomères.

13. Composition selon l'une quelconque des revendications 4 à 12, **caractérisée par le fait que** le polymère siliconé greffé a un poids moléculaire moyen en nombre allant de 10.000 à 2.000.000 et une température de transition vitreuse Tg ou une température de fusion cristalline Tm d'au moins -20°C.

14. Composition selon l'une quelconque des revendications 1 à 3, **caractérisée par le fait que** le polymère siliconé greffé est un copolymère susceptible d'être obtenu par extrusion réactive d'un macromère polysiloxane ayant une fonction réactive terminale sur un polymère du type polyoléfine comportant des groupes réactifs susceptibles de réagir avec la fonction réactive terminale du macromère polysiloxane pour former une liaison covalente permettant le greffage de la silicone sur la chaîne principale de la polyoléfine.

15. Composition selon la revendication 14, **caractérisée par le fait que** la polyoléfine réactive est choisie dans le groupe constitué par les polyéthylènes ou les polymères de monomères dérivés de l'éthylène comportant des fonctions réactives susceptibles de réagir avec la fonction terminale du macromère polysiloxane.

16. Composition selon la revendication 14 ou 15, **caractérisée par le fait que** la polyoléfine réactive est choisie dans le groupe constitué par les copolymères d'ethylène ou de dérivés d'éthylène et de monomères choisis parmi ceux comportant une fonction carboxylique ; ceux comportant une fonction anhydride d'acide ; ceux comportant une fonction chlorure d'acide ; ceux comportant une fonction ester ; ceux comportant une fonction isocyanate.

17. Composition selon l'une quelconque des revendications 14 à 16, **caractérisée par le fait que** le macromère polysiloxane est un polysiloxane comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires.

18. Composition selon l'une quelconque des revendications 14 à 17, **caractérisée par le fait que** le macromère polysiloxane est un polysiloxane répondant à la formule générale (III):
T-(CH₂)ₛ-Si-[-(OSiR⁵R⁶)ₜ-R⁷]_{y} (III)
dans laquelle T est choisi dans le groupe constitué par NH2, NHR', une fonction époxy, OH, SH ; R⁵, R⁶, R⁷ et R', indépendamment, désignent un alkyle en C₁-C₆, phényle, benzyle, ou alkylphényle en C₆-C₁₂, hydrogène ; s est un nombre de 2 à 100 ; t est un nombre de 0 à 1000 et y est un nombre de 1 à 3.

19. Composition selon l'une quelconque des revendications 1 à 18, **caractérisée par le fait que** le polymère siliconé greffé est utilisé en une quantité allant de 0,01 à 20% en poids par rapport au poids total de la composition et de préférence de 0,1 à 15% en poids et encore plus préférentiellement de 0,5 à 10% en poids.

20. Composition selon l'une quelconque des revendications 1 à 19, **caractérisée par le fait que** les polymères ioniques amphiphiles comportant au moins une chaîne grasse et au moins un motif hydrophile sont utilisés en une quantité allant de 0,01 à 20% en poids du poids total de la composition et plus préférentiellement de 0,1 à 15% en poids et encore plus préférentiellement de 0,5 à 10% en poids.

21. Composition selon l'une quelconque des revendications 1 à 20, **caractérisée par le fait qu'**elle contient en plus au moins un additif choisi dans le groupe constitué par les épaississants sans chaîne grasse, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

22. Composition selon l'une quelconque des revendications 1 à 21, **caractérisée par le fait que** le milieu cosmétiquement ou dermatologiquement acceptable est constitué par de l'eau ou un mélange d'eau et d'au moins un solvant cosmétiquement acceptable.

23. Composition selon la revendication 22, **caractérisée par le fait que** les solvants cosmétiquement acceptables sont choisis dans le groupe constitué par les monoalcools, les polyalcools, les éthers de glycol, les esters d'acides gras et leurs mélanges.

24. Composition selon l'une quelconque des revendications 1 à 23, **caractérisée par le fait que** les matières kératiniques sont des cheveux humains.

25. Composition selon l'une quelconque des revendications 1 à 24, **caractérisée par le fait que** le polymère siliconé greffé est dissous dans le milieu cosmétiquement ou dermatologiquement acceptable ou utilisé sous forme de dispersion aqueuse de particules.

26. Composition selon l'une quelconque des revendications 1 à 25, **caractérisée par le fait qu'**elle se présente sous forme de gel, de lait, de crème, de lotion plus ou moins épaissie ou de mousse.

27. Composition selon l'une quelconque des revendications 1 à 26, **caractérisée par le fait qu'**elle est un produit de coiffage.

28. Composition selon l'une quelconque des revendications 1 à 27, **caractérisée par le fait qu'**elle est un produit capillaire choisi dans le groupe constitué par des shampooings; des produits capillaires à rincer ou non, à appliquer avant ou après un shampooing, une coloration, une décoloration, une permanente ou un défrisage.

29. Composition selon l'une quelconque des revendications 1 à 28, **caractérisée par le fait qu'**elle est conditionnée sous forme de vaporisateur, de flacon pompe ou bien dans un récipient aérosol en vue d'obtenir un spray, une laque ou une mousse.

30. Procédé non-thérapeutique de traitement des matières kératiniques, en particulier des cheveux humains, **caractérisé par le fait qu'**il consiste à appliquer sur lesdites matières une composition telle que définie selon l'une quelconque des revendications 1 à 29 puis à effectuer éventuellement un rinçage à l'eau.

## Patentansprüche

1. Kosmetische oder dermatologische Zusammensetzung, die zur Behandlung von Keratinsubstanzen vorgesehen ist, **dadurch gekennzeichnet, daß** sie in einem kosmetisch oder dermatologisch akzeptablen Medium mindestens ein gepfropftes Siliconpolymer mit einem nicht siliconhaltigen, organischen Grundgerüst, auf das Monomere gepfropft sind, die ein Polysiloxan enthalten, und mindestens ein amphiphiles ionisches Polymer enthält, das mindestens eine Fettkette und mindestens eine hydrophile Einheit aufweist und ausgewählt ist unter:
- ionischen Holosiden, die mit Gruppen modifiziert sind, die mindestens eine Fettkette enthalten;
- Copolymeren von Maleinsäureanhydrid und Monomeren, die mindestens eine Fettkette aufweisen;
- Vinylacetat/Crotonsäure/Allylstearat-Terpolymeren;
- vernetzten Acrylsäure/Vinylisodecanoat-Copolymeren;
- Acrylsäure/Vinylpyrrolidon/Laurylmethacrylat-Terpolymeren;
- Acrylsäure/Lauryl(meth)acrylat-Copolymeren;
- Methacrylsäure/Ethylacrylat/polyethoxylierter Stearylallylether-Terpolymeren;
- Methacrylsäure/Ethylacrylat/polyethoxyliertes Laurylacrylat-Terpolymeren;
- Methacrylsäure/Ethylacrylat/polyethoxyliertes Stearylmethacrylat-Terpolymeren;
- Methacrylsäure/ Ethylacrylat/polyethoxyliertes Nonylphenolacrylat-Copolymeren;
- Acrylsäure/polyethoxyliertes Stearylmonoitaconat-Copolymeren;
- Acrylsäure/polyethoxyliertes Cetylmonoitaconat-Copolymeren;
- Copolymeren von Methacrylsäure, Butylacrylat und einem hydrophoben Monomer mit Fettkette;
- Terpolymeren von Acrylsäure/C₁₅-Alkylacrylat/Polyethylenglykolacrylat (28 mol Ethylenoxid);
- partiellen Fettsäureestersalzen eines Copolymers von Acrylsäure/Dimethylethanolamin;
- Copolymeren von Acrylsäure, Acrylat und einem amphiphilen Monomer, das eine Fettkette mit Urethangruppen aufweist.

2. Zusammensetzung nach Anspruch 1, **dadurch gekennzeichnet, daß** das gepfropfte Siliconpolymer aus einer organischen Hauptkette besteht, die aus organischen Monomeren gebildet wird, die kein Silicon enthalten, auf die in der Kette sowie gegebenenfalls an mindestens einem ihrer Enden ein Polysiloxanmakromer gepfropft ist.

3. Zusammensetzung nach Anspruch 2, **dadurch gekennzeichnet, daß** die nicht siliconhaltigen, organischen Monomere, die die Hauptkette des gepfropften Siliconpolymers bilden, unter den auf radikalischem Wege polymerisierbaren Monomeren mit ethylenischer Doppelbindung, den durch Polykondensation polymerisierbaren Monomeren und den unter Ringöffnung polymerisierbaren Monomeren ausgewählt sind.

4. Zusammensetzung nach einem der Ansprüche 1 bis 3, **dadurch gekennzeichnet, daß** das gepfropfte Siliconpolymer ein gepfropftes Siliconcopolymer ist, das enthält:
a) 0 bis 98 Gew.-% mindestens eines lipophilen Monomers (A) von geringer Polarität mit ethylenischer Doppelbindung, das radikalisch polymerisierbar ist;
b) 0 bis 98 Gew.-% mindestens eines polaren hydrophilen Monomers (B) mit ethylenischer Doppelbindung, das mit dem (den) Monomer(en) vom Typ (A) copolymerisierbar ist;
c) 0,01 bis 50 Gew.-% mindestens eines Polysiloxanmakromers (C) der allgemeinen Formel:
X(Y)ₙSi(R)₃₋ₘZₘ (I)
worin bedeuten:
- X eine mit den Monomeren (A) und (B) copolymerisierbare Vinylgruppe;
- Y eine zweiwertige Verbindungsgruppe;
- R Wasserstoff, Alkyl oder Alkoxy mit 1 bis 6 Kohlenstoffatomen, C₆₋₁₂-Aryl;
- Z eine einwertige Polysiloxaneinheit mit einem Zahlenmittel des Molekulargewichts von mindestens 500;
- n 0 oder 1 und m eine ganze Zahl von 1 bis 3; wobei die Prozentzahlen bezogen auf das Gesamtgewicht der Monomeren (A), (B) und (C) angegeben sind.

5. Zusammensetzung nach Anspruch 4, **dadurch gekennzeichnet, daß** die lipophilen Monomere (A) unter Estern von Acrylsäure oder Methacrylsäure und C₁₋₁₈-Alkoholen; Styrol; Polystyrolmakromeren; Vinylacetat; Vinylpropionat; α-Methylstyrol; tert.-Butylstyrol; Butadien; Cyclohexadien; Ethylen; Propylen; Vinyltoluol; Estern von Acrylsäure oder Methacrylsäure und 1,1-Dihydroperfluoralkanolen oder ihren homologen Verbindungen; Estern von Acrylsäure oder Methacrylsäure und omega-Hydridofluoralkanolen; Estern von Acrylsäure oder Methacrylsäure und Fluoralkylsulfonamidoalkoholen; Estern von Acrylsäure oder Methacrylsäure und Fluoralkylalkoholen; Estern von Acrylsäure oder Methacrylsäure und Fluorethern von Alkoholen; oder deren Gemischen ausgewählt sind.

6. Zusammensetzung nach Anspruch 5, **dadurch gekennzeichnet, daß** die lipophilen Monomere (A) unter n-Butylmethacrylat, Isobutylmethacrylat, tert.-Butylacrylat, tert.-Butylmethacrylat, 2-Ethylhexylmethacrylat, Methylmethacrylat, 2-(N-Butyl-perfluoroctan-sulfonamido)-ethylacrylat; und 2-(N-Methyl-perfluoroctan-sulfonamido)-ethylacrylat ausgewählt sind.

7. Zusammensetzung nach einem der Ansprüche 4 bis 6, **dadurch gekennzeichnet, daß** die polaren Monomere (B) unter Acrylsäure, Methacrylsäure, N,N-Dimethylacrylamid, Dimethylaminoethylmethacrylat, quatemisiertem Dimethylaminoethylmethacrylat, (Meth)acrylamid, N-t-Butyl-acrylamid, Maleinsäure, Maleinsäureanhydrid und seinen Halbestern, hydroxyalkylierten (Meth)acrylaten, Diallyldimethylammoniumchlorid, Vinylpyrrolidon, Vinylethern, Maleimiden, Vinylpyridin, Vinylimidazol, heterocyclischen polaren Vinylverbindungen, Styrolsulfonat, Allylalkohol, Vinylalkohol, Vinylcaprolactam oder deren Gemischen ausgewählt sind.

8. Zusammensetzung nach Anspruch 7, **dadurch gekennzeichnet, daß** die polaren Monomere (B) unter Acrylsäure, N,N-Dimethylacrylamid, Dimethylaminoethylmethacrylat, quaternisiertem Dimethylaminoethylmethacrylat, Vinylpyrrolidon und deren Gemischen ausgewählt sind.

9. Zusammensetzung nach einem der Ansprüche 4 bis 8, **dadurch gekennzeichnet, daß** das Polysiloxanmakromer (C) der folgenden allgemeinen Formel (II) entspricht: 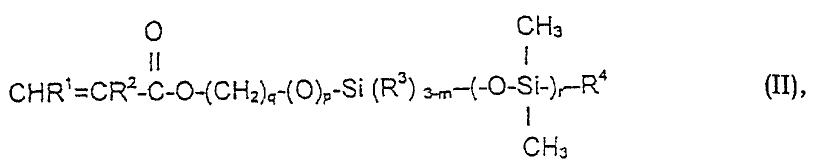 worin bedeuten:
R¹ Wasserstoff oder -COOH,
R² Wasserstoff, Methyl oder -CH₂COOH,
R³ Alkyl, Alkoxy oder Alkylamino mit 1 bis 6 Kohlenstoffatomen, C₆₋₁₂-Aryl oder Hydroxy,
R⁴ Alkyl, Alkoxy oder Alkylamino mit 1 bis 6 Kohlenstoffatomen, C₆₋₁₂-Aryl oder Hydroxy,
q eine ganze Zahl von 2 bis 6,
p 0 oder 1,
r eine ganze Zahl von 5 bis 700,
m eine ganze Zahl von 1 bis 3.

10. Zusammensetzung nach einem der Ansprüche 4 bis 9, **dadurch gekennzeichnet, daß** das Polysiloxanmakromer (C) der folgenden allgemeinen Formel entspricht: 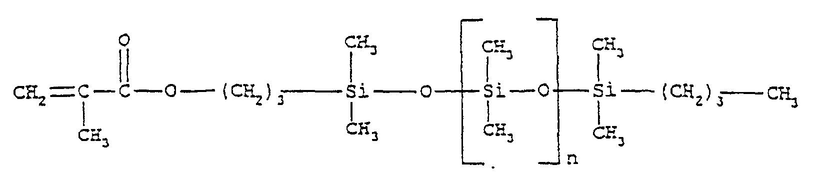 wobei n eine Zahl im Bereich von 5 bis 700 bedeutet, wobei die Prozentzahlen bezogen auf das Gesamtgewicht der Monomere angegeben sind

11. Zusammensetzung nach einem der Ansprüche 1 bis 10, **dadurch gekennzeichnet, daß** das gepfropfte Siliconpolymer ein Copolymer ist, das durch radikalische Polymerisation ausgehend von folgendem Monomerengemisch erhältlich ist:
a. 60 Gew.-% tert.-Butylacrylat,
b. 20 Gew.-% Acrylsäure,
c. 20 Gew.-% Siliconmakromer der Formel: 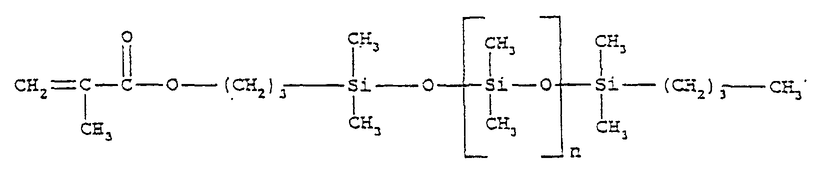
wobei n eine Zahl von 5 bis 700 ist, wobei die Prozentzahlen bezogen auf das Gesamtgewicht der Monomere angegeben sind.

12. Zusammensetzung nach einem der Ansprüche 1 bis 10, **dadurch gekennzeichnet, daß** das gepfropfte Siliconpolymer ein Copolymer ist, das durch radikalische Polymerisation ausgehend von folgendem Monomerengemisch erhältlich ist:
a) 80 Gew.-% tert.-Butylacrylat,
b) 20 Gew.-% Siliconmakromer der Formel: 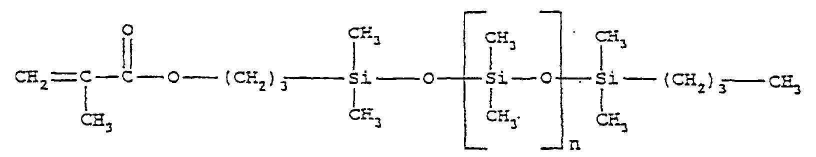
wobei n eine Zahl von 5 bis 700 ist; die Prozentzahlen sind bezogen auf das Gesamtgewicht der Monomere angegeben.

13. Zusammensetzung nach einem der Ansprüche 4 bis 12, **dadurch gekennzeichnet, daß** das gepfropfte Siliconpolymer ein Zahlenmittel des Molekulargewichts von 10 000 bis 2 000 000 und eine Glasübergangstemperatur T_{g} oder eine kristalline Schmelztemperatur Tₘ von mindestens -20 °C aufweist.

14. Zusammensetzung nach einem der Ansprüche 1 bis 3, **dadurch gekennzeichnet, daß** das gepfropfte Siliconpolymer ein Copolymer ist, das durch reaktive Extrusion eines Polysiloxanmakromers mit einer reaktiven Endgruppe und eines Polymers vom Polyolefintyp erhältlich ist, das Gruppen aufweist, die mit der reaktiven Endgruppe des Polysiloxanmakromers reagieren können, wodurch eine kovalente Bindung ausgebildet wird, über die das Silicon auf die Polyolefin-Hauptkette gepfropft werden kann.

15. Zusammensetzung nach Anspruch 14, **dadurch gekennzeichnet, daß** das reaktive Polyolefin unter den Polyethylenen oder den Polymeren von Monomeren, die von Ethylen abgeleitet sind, ausgewählt sind, die reaktive Gruppen aufweisen, die mit der Endgruppe des Polysiloxanmakromers reagieren können.

16. Zusammensetzung nach Anspruch 14 oder 15, **dadurch gekennzeichnet, daß** das reaktive Polyolefin unter den Copolymeren von Ethylen oder Derivaten von Ethylen und Monomeren, die eine Carboxygruppe aufweisen; Monomeren, die eine Anhydridgruppe aufweisen; Monomeren, die ein Säurechloridgruppe enthalten; Monomeren, die eine Estergruppe aufweisen; und Monomeren, die eine Isocyanatgruppe enthalten, ausgewählt sind.

17. Zusammensetzung nach einem der Ansprüche 14 bis 16, **dadurch gekennzeichnet, daß** das Polysiloxanmakromer ein Polysiloxan ist, das am Ende der Polysiloxankette oder in der Nähe des Kettenendes eine funktionelle Gruppe aufweist, die unter Alkoholen, Thiolen, Epoxy und primären und sekundären Aminen ausgewählt ist.

18. Zusammensetzung nach einem der Ansprüche 14 bis 17, **dadurch gekennzeichnet, daß** das Polysiloxanmakromer ein Polysiloxan ist, das der folgenden allgemeinen Formel (III) entspricht:
T-(CH₂)ₛ-Si-[-(OSiR⁵R⁶)ₜ-R⁷]_{y} (III),
worin T unter NH₂, NHR', Epoxy, OH und SH ausgewählt ist; R⁵, R⁶, R⁷ und R' unabhängig voneinander C₁₋₆-Alkyl, Phenyl, Benzyl, C₆₋₁₂-Alkylphenyl oder Wasserstoff bedeuten; s eine Zahl von 2 bis 100; t eine Zahl von 0 bis 1 000; und y eine Zahl von 1 bis 3 ist.

19. Zusammensetzung nach einem der Ansprüche 1 bis 18, **dadurch gekennzeichnet, daß** das gepfropfte Siliconpolymer in Konzentrationen von 0,01 bis 20 Gew.-%, bezogen auf das Gesamtgewicht der Zusammensetzung, vorzugsweise im Bereich von 0,1 bis 15 Gew.-% und insbesondere im Bereich von 0,5 bis 10 Gew.-% verwendet wird.

20. Zusammensetzung nach einem der Ansprüche 1 bis 19, **dadurch gekennzeichnet, daß** die ionischen amphiphilen Polymere, die mindestens eine Fettkette und mindestens eine hydrophobe Einheit aufweisen, in einem Mengenanteil von 0,01 bis 20 Gew.-%, bezogen auf das Gesamtgewicht der Zusammensetzung, vorzugsweise 0,1 bis 15 Gew.-% und noch bevorzugter 0,5 bis 10 Gew.-% verwendet werden.

21. Zusammensetzung nach einem der Ansprüche 1 bis 20, **dadurch gekennzeichnet, daß** sie ferner mindestens einen Zusatzstoff enthält, der unter den Verdickungsmitteln ohne Fettkette, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

22. Zusammensetzung nach einem der Ansprüche 1 bis 21, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

23. Zusammensetzung nach Anspruch 22, **dadurch gekennzeichnet, daß** die kosmetisch akzeptablen Lösungsmittel unter Monoalkoholen, Polyalkoholen, Glykolethern, Fettsäureestern und deren Gemischen ausgewählt sind.

24. Zusammensetzung nach einem der Ansprüche 1 bis 23, **dadurch gekennzeichnet, daß** es sich bei den Keratinsubstanzen um menschliches Haar handelt.

25. Zusammensetzung nach einem der Ansprüche 1 bis 24, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

26. Zusammensetzung nach einem der Ansprüche 1 bis 25, **dadurch gekennzeichnet, daß** sie als Gel, Milch, Creme, mehr oder weniger dickflüssige Lotion oder Schaum vorliegt.

27. Zusammensetzung nach einem der Ansprüche 1 bis 26, **dadurch gekennzeichnet, daß** es sich um ein Produkt zum Frisieren handelt.

28. Zusammensetzung nach einem der Ansprüche 1 bis 27, **dadurch gekennzeichnet, daß** es sich um ein Produkt für das Haar handelt, das unter den Haarwaschmitteln und Produkten für das Haar ausgewählt ist, die ausgespült oder nicht ausgespült werden und vor oder nach einer Haarwäsche, Färbung, Entfärbung, Dauerwelle oder Entkräuselung aufgetragen werden.

29. Zusammensetzung nach einem der Ansprüche 1 bis 28, **dadurch gekennzeichnet, daß** sie in einem Zerstäuber, Pumpflakon oder Aerosolbehälter konfektioniert ist, um ein Spray, einen Lack oder einen Schaum zu erhalten.

30. Nicht therapeutisches Verfahren zur Behandlung von Keratinsubstanzen und insbesondere menschlichem Haar, **dadurch gekennzeichnet, daß** es darin besteht, auf die Keratinsubstanzen eine Zusammensetzung nach einem der Ansprüche 1 bis 29 aufzutragen und gegebenenfalls mit Wasser zu spülen.

## Claims

1. Cosmetic or dermatological composition intended for treating keratin materials, **characterized in that** it contains, in a cosmetically or dermatologically acceptable medium, at least one grafted silicone polymer with a non-silicone organic skeleton grafted with monomers containing a polysiloxane and at least one ionic amphiphilic polymer comprising at least one fatty chain and at least one hydrophilic unit, chosen from:
- ionic holosides modified with groups comprising at least one fatty chain;
- copolymers of maleic anhydride and of monomers comprising at least one fatty chain;
- vinyl acetate/crotonic acid/allyl stearate terpolymers ;
- acrylic acid/vinyl isodecanoate crosslinked copolymers;
- acrylic acid/vinylpyrrolidone/lauryl methacrylate terpolymers;
- acrylic acid/lauryl (meth)acrylate copolymers;
- methacrylic acid/ethyl acrylate/polyethoxylated stearyl allyl ether terpolymers;
- methacrylic acid/ethyl acrylate/polyoxyethylenated lauryl acrylate terpolymers;
- methacrylic acid/ethyl acrylate/polyoxyethylenated stearyl methacrylate terpolymers;
- methacrylic acid/ethyl acrylate/polyoxyethylenated nonylphenyl acrylate copolymers;
- acrylic acid/polyoxyethylenated stearyl monoitaconate copolymers;
- acrylic acid/polyoxyethylenated cetyl monoitaconate copolymers;
- copolymers of methacrylic acid/butyl acrylate/ hydrophobic monomer comprising a fatty chain;
- acrylic acid/C₁₅ alkyl acrylate/polyethylene glycol acrylate (28 mol of ethylene oxide) terpolymers;
- salts of a partial fatty acid ester of an acrylic acid/dimethylethanolamine copolymer;
- copolymers of acrylic acid/acrylate/amphiphilic monomer comprising a fatty chain containing urethane groups.

2. Composition according to Claim 1, **characterized in that** the grafted silicone polymer consists of an organic main chain formed from organic monomers containing no silicone, onto which are grafted, within the said chain and also optionally on at least one of its ends, at least one polysiloxane macromer.

3. Composition according to Claim 2, **characterized in that** the non-silicone organic monomers constituting the main chain of the grafted silicone polymer are chosen from the group consisting of monomers containing ethylenic unsaturation which are polymerizable by free-radical polymerization, monomers which are polymerizable by polycondensation and monomers which undergo ring opening.

4. Composition according to any one of Claims 1 to 3, **characterized in that** the grafted silicone polymer is a silicone grafted copolymer comprising:
a) from 0% to 98% by weight of at least one lipophilic monomer (A) of low polarity containing ethylenic unsaturation of low polarity, which is polymerizable by free-radical polymerization;
b) from 0% to 98% by weight of at least one polar hydrophilic monomer (B) containing ethylenic unsaturation, which is copolymerizable with the monomer(s) of the type (A);
c) from 0.01% to 50% by weight of at least one polysiloxane macromer (C) of general formula:
X(Y)ₙSi(R)₃₋ₘZₘ (I)
where:
X denotes a vinyl group which is copolymerizable with the monomers (A) and (B) ;
Y denotes a divalent bonding group;
R denotes a hydrogen, a C₁-C₆ alkyl or alkoxy or a C₆-C₁₂ aryl;
Z denotes a monovalent polysiloxane unit with a number-average molecular weight of at least 500;
n is 0 or 1 and m is an integer ranging from 1 to 3; the percentages being calculated relative to the total weight of the monomers (A), (B) and (C).

5. Composition according to Claim 4, **characterized in that** the lipophilic monomers (A) are chosen from the group consisting of acrylic or methacrylic acid esters of C₁-C₁₈ alcohols; styrene; polystyrene macromers; vinyl acetate; vinyl propionate; α-methylstyrene; tert-butylstyrene; butadiene; cyclohexadiene; ethylene; propylene; vinyltoluene; acrylic or methacrylic acid esters of 1,1-dihydroperfluoroalkanol or homologues thereof; acrylic or methacrylic acid esters of ω-hydrofluoroalkanol; acrylic or methacrylic acid esters of fluoroalkylsulphonamido-alcohol; acrylic or methacrylic acid esters of fluoroalkyl alcohol; acrylic or methacrylic acid esters of fluoroether alcohol; or mixtures thereof.

6. Composition according to Claim 5, **characterized in that** the lipophilic monomers (A) are chosen from the group consisting of n-butyl methacrylate, isobutyl methacrylate, tert-butyl acrylate, tert-butyl methacrylate, 2-ethylhexyl methacrylate, methyl methacrylate, 2-(N-butylperfluorooctanesulphonamido)ethyl acrylate and 2- (N-methylperfluorooctanesulphonamido) ethyl acrylate, and mixtures thereof.

7. Composition according to any one of Claims 4 to 6, **characterized in that** the polar monomers (B) are chosen from the group consisting of acrylic acid, methacrylic acid, N,N-dimethylacrylamide, dimethylaminoethyl methacrylate, quaternized dimethylaminoethyl methacrylate, (meth)acrylamide, N-t-butylacrylamide, maleic acid, maleic anhydride and partial esters thereof, hydroxyalkyl (meth)acrylates, diallyldimethylammonium chloride, vinylpyrrolidone, vinyl ethers, maleimides, vinylpyridine, vinylimidazole, heterocyclic vinyl polar compounds, styrene sulphonate, allylic alcohol, vinyl alcohol and vinyl caprolactam, or mixtures thereof.

8. Composition according to Claim 7, **characterized in that** the polar monomers (B) are chosen from the group consisting of acrylic acid, N,N-dimethylacrylamide, dimethylaminoethyl methacrylate, quaternized dimethylaminoethyl methacrylate and vinylpyrrolidone, and mixtures thereof.

9. Composition according to any one of Claims 4 to 8, **characterized in that** the polysiloxane macromer (C) corresponds to the general formula (II) below: 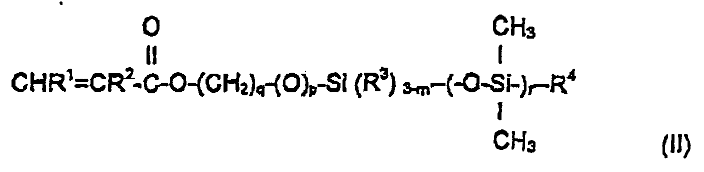 in which:
R¹ is hydrogen or -COOH;
R² is hydrogen, methyl or -CH₂COOH;
R³ is C₁-C₆ alkyl, alkoxy or alkylamino, C₆-C₁₂ aryl or hydroxyl;
R⁴ is C₁-C₆ alkyl, alkoxy or alkylamino, C₆-C₁₂ aryl or hydroxyl;
q is an integer from 2 to 6;
p is 0 or 1;
r is an integer from 5 to 700;
m is an integer from 1 to 3.

10. Composition according to any one of Claims 4 to 9, **characterized in that** the polysiloxane macromer (C) corresponds to the following general formula: 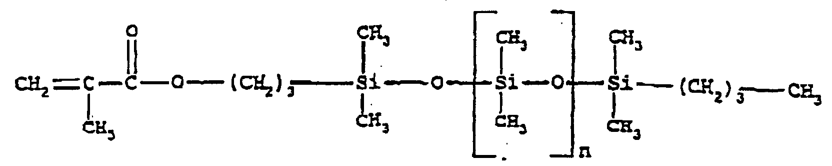 with n being a number ranging from 5 to 700; the percentages by weight being calculated relative to the total weight of the monomers.

11. Composition according to any one of Claims 1 to 10, **characterized in that** the grafted silicone polymer is a copolymer which may be obtained by free-radical polymerization starting with a monomer mixture consisting of:
a) 60% by weight of tert-butyl acrylate;
b) 20% by weight of acrylic acid;
c) 20% by weight of silicone macromer of formula: 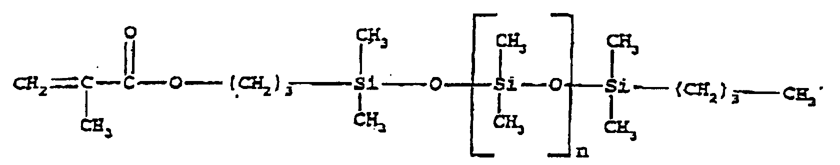 with n being a number ranging from 5 to 700; the percentages by weight being calculated relative to the total weight of the monomers.

12. Composition according to any one of Claims 1 to 10, **characterized in that** the grafted silicone polymer is a copolymer which may be obtained by free-radical polymerization starting with a monomer mixture consisting of:
a) 80% by weight of tert-butyl acrylate;
b) 20% by weight of silicone macromer of formula : 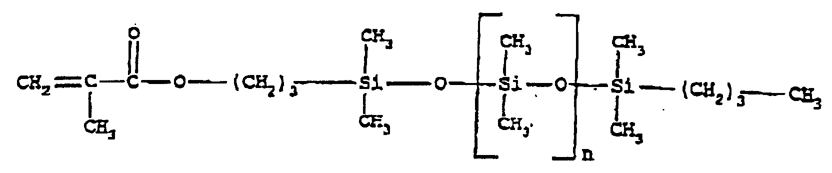 with n being a number ranging from 5 to 700; the percentages by weight being calculated relative to the total weight of the monomers.

13. Composition according to any one of Claims 4 to 12, **characterized in that** the grafted silicone polymer has a number-average molecular weight ranging from 10 000 to 2 000 000 and a glass transition temperature Tg or a crystal melting point Tm of at least -20°C.

14. Composition according to any one of Claims 1 to 3, **characterized in that** the grafted silicone polymer is a copolymer which may be obtained by reactive extrusion of a polysiloxane macromer containing a reactive end function with a polymer of the polyolefin type comprising reactive groups capable of reacting with the reactive end function of the polysiloxane macromer to form a covalent bond for grafting the silicone to the main chain of the polyolefin.

15. Composition according to Claim 14, **characterized in that** the reactive polyolefin is chosen from the group consisting of polyethylenes and polymers of monomers derived from ethylene comprising reactive functions capable of reacting with the end function of the polysiloxane macromer.

16. Composition according to Claim 14 or 15, **characterized in that** the reactive polyolefin is chosen from the group consisting of copolymers of ethylene or of ethylene derivatives and of monomers chosen from those comprising a carboxylic function; those comprising an acid anhydride function; those comprising an acid chloride function; those comprising an ester function; those comprising an isocyanate function.

17. Composition according to any one of Claims 14 to 16, **characterized in that** the polysiloxane macromer is a polysiloxane comprising a functionalized group, at the end of the polysiloxane chain or close to the end of the said chain, chosen from the group consisting of alcohols, thiols, epoxy groups and primary and secondary amines.

18. Composition according to any one of Claims 14 to 17, **characterized in that** the polysiloxane macromer is a polysiloxane corresponding to the general formula (III):
T-(CH₂)ₛ-Si-[-(OSiR⁵R⁶)ₜ-R7]_{y} (III)
in which T is chosen from the group consisting of NH₂, NHR' and an epoxy, OH or SH function; R⁵, R⁶, R⁷ and R' independently denote a C₁-C₆ alkyl, phenyl, benzyl or C₆-C₁₂ alkylphenyl, or hydrogen; s is a number from 2 to 100; t is a number from 0 to 1 000 and y is a number from 1 to 3.

19. Composition according to any one of Claims 1 to 18, **characterized in that** the grafted silicone polymer is used in an amount ranging from 0.01% to 20% by weight relative to the total weight of the composition, preferably from 0.1% to 15% by weight and even more preferably from 0.5% to 10% by weight.

20. Composition according to any one of Claims 1 to 19, **characterized in that** the amphiphilic ionic polymers comprising at least one fatty chain and at least one hydrophilic unit are used in an amount ranging from 0.01% to 20% by weight relative to the total weight of the composition, more preferably from 0.1% to 15% by weight and even more preferably from 0.5% to 10% by weight.

21. Composition according to any one of Claims 1 to 20, **characterized in that** it also contains at least one additive chosen from the group consisting of fatty-chain-free thickeners, fatty acid esters, fatty acid esters of glycerol, silicones, surfactants, fragrances, preserving agents, sunscreens, proteins, vitamins, polymers, plant, animal, mineral or synthetic oils and any other additive conventionally used in cosmetics.

22. Composition according to any one of Claims 1 to 21, **characterized in that** the cosmetically or dermatologically acceptable medium consists of water or a mixture of water and at least one cosmetically acceptable solvent.

23. Composition according to Claim 22, **characterized in that** the cosmetically acceptable solvents are chosen from the group consisting of monoalcohols, polyalcohols, glycol ethers and fatty acid esters, and mixtures thereof.

24. Composition according to any one of Claims 1 to 23, **characterized in that** the keratin materials are human hair.

25. Composition according to any one of Claims 1 to 24, **characterized in that** the grafted silicone polymer is dissolved in the cosmetically or dermatologically acceptable medium or used in the form of an aqueous particle dispersion.

26. Composition according to any one of Claims 1 to 25, **characterized in that** it is in the form of a gel, a milk, a cream, a more or less thickened lotion or a mousse.

27. Composition according to any one of Claims 1 to 26, **characterized in that** it is a styling product.

28. Composition according to any one of Claims 1 to 27, **characterized in that** it is a haircare product chosen from the group consisting of shampoos; and rinse-out or leave-in hair products, to be applied before or after shampooing, dyeing, bleaching, permanent-waving or relaxing the hair.

29. Composition according to any one of Claims 1 to 28, **characterized in that** it is packaged in the form of a vaporizer, a pump-dispenser bottle or in an aerosol container in order to obtain a spray, a lacquer or a mousse.

30. Non-therapeutic process for treating keratin materials, in particular human hair, **characterized in that** it consists in applying a composition as defined according to any one of Claims 1 to 29 to the said materials and then in optionally rinsing with water.
